# EUROPEAN PATENT APPLICATION

(11) **EP 3 457 405 A1**
(43) Date of publication of application: **20.03.2019**
(21) Application number: 17191405.4
(22) Date of filing: 15.09.2017
(51) Int. Cl.: G16H 30/40, A61B 6/00, A61B 5/05, G06K 9/46, G06T 7/00, G06K 9/66, G16H 40/60, G16H 50/70

(54) **METHOD FOR IDENTIFYING AN EXOGENOUS STRUCTURE, SYSTEM FOR IDENTIFYING AN EXOGENOUS STRUCTURE, COMPUTER PROGRAM PRODUCT AND COMPUTER-READABLE MEDIUM**

(71) Applicant: Siemens Healthcare GmbH, 91052 Erlangen (DE)
(72) Inventor: Schmidt, Sebastian, 91085 Weisendorf (DE); Grodzki, David, 91058 Erlangen (DE); Kuth, Rainer, 91315 Höchstadt (DE); Heitland, Axel, 91054 Erlangen (DE)

(57) **Abstract**

Method for identifying an exogenous structure, system for identifying an exogenous structure, computer program product and computer-readable medium

The present invention suggests a method for identifying an exogenous structure, in particular an implant, a prosthesis or metal residues, in a medical imaging data set being recorded by a medical imaging device, comprising:
- providing a database classifying exogenous structures by a reference parameter set;
- providing the medical imaging data set;
- extracting an image parameter set from the medical imaging data set; and
- assigning the reference parameter set to the image parameter set for identifying the exogenous structure.

## Description

The present invention describes a method for identifying an exogenous structure, a system for identifying an exogenous structure, a computer program product and a computer-readable medium.

Implants, prostheses or metal residues are well known exogenous structures that might be located inside a body of a patient. The properties of these exogenous structures determine whether a medical imaging device can be used for recording a medical image or how the medical imaging device has to be configured. However, patients typically do not know exact specifics about their implants or prosthesis, in particular essential specifics about maintenance or malfunctions that become known after the implant or prosthesis has been incorporated. Further, medical records of the patient that might include information about the exogenous structure might be not updated or available in an emergency situation.

Therefore, it is an object of the present invention to provide a method for identifying an exogenous structure and in particular to support an operator of a medical imaging device by identifying the exogenous structure.

This object is achieved by the method according to claim 1 for identifying an exogenous structure, by the system according to claim 13, the computer program product according to claim 14 and by the computer readable computer medium according to claim 15.

According to a first aspect of the present invention, a method for identifying an exogenous structure, in particular an implant, a prosthesis or metal residues, in a medical imaging data set being recorded by a medical imaging device, is provided, comprising:
- providing a database classifying exogenous structures by a reference parameter set;
- providing the medical imaging data set;
- extracting an image parameter set from the medical imaging data set; and
- assigning the reference parameter set to the image parameter set for identifying the exogenous structure.

In contrast to the state of the art, it is advantageously possible to identify the exogenous structure without relying on information of the patient and/or the medical record of the patient. Thus, a probability of damages caused by using the improper medical imaging device can be reduced, and/or a configuration of a setting of the medical imaging device can be optimized based on the identification for improving the quality of the visualized medical image data set. Further, an operator of the medical imaging device can inform a patient or a colleague about the specifics of the exogenous structures, in particular about maintenance or malfunctions that become known after the implant or prosthesis has been incorporated. For example a pacemaker can be identified, such as a dual-chamber pacemaker including an atrial electrode and main electrode. Preferably, it is provided that the method does not only identify the kind of exogenous structure but also a specific type of the respective exogenous structure, such as a pacemaker, including information about e.g. manufacturer, model, type, etc. In other words: the method does not only differentiate between implants but also between different implants of the same kind, for example by specifying a producer or a line of implants manufactured by the producer. For assigning the reference parameter set to the image data set, the reference parameter set and the image data set do not need match exactly. It is also thinkable that the reference parameter set and the image data set match within a tolerance limit. Thereby, it is preferably provided that an evaluation unit is provided for assigning the reference parameter set to the image parameter set. Such an evaluation unit might comprises a processor being configured for assigning the reference parameter set to the image data set. Furthermore, it is conceivable that the reference parameter set is greater than the image data set, i.e., the amount of information of the reference parameter set is greater than the amount of information of the image data set, and the image data set is assigned to a subset of the reference parameter set.

In general, the term "exogenous structure" describes all artificial or man-made structures that might be implanted into a body of a patient. These exogenous structures might be implanted on purpose, such as implants or prostheses, or by accident, such as metal residues being inside the body due to a gunshot wound, splitter or residues from a surgery. In particular, the term "exogenous structures" represents all structures being not produced naturally in the body and might cause damages or artefacts during recording a medical image by the medical imaging device. The Medical imaging device might be an x-ray-imaging device, a computer tomography (CT) device, a magnetic resonance tomography (MRT) device or an ultrasound scanner. Further the terms "reference parameter set", "medical imaging data set" and "image parameter set" represent data sets such as vectors, two-dimensional, three-dimensional and/or four-or more-dimensional data sets.

Preferably, an evaluation unit comprising a processor is provided wherein the processor is configured for extracting an image parameter set from the medical imaging data set and assigning the reference parameter set to the image parameter set for identifying the exogenous structure. In particular, the processor is part of a workstation or part of a network. For example, the processor is included in a server or a system of servers. It is also thinkable that at least one step of the method according to the present invention is performed by a server or a system of servers. Thereby a workstation and/or the medical imaging device are in communication with each other.

Particularly advantageous embodiments and features of the invention are given by the dependent claims as revealed in the following description. Features of different claim categories may be combined as appropriate to give further embodiments not described herein.

According to a preferred embodiment of the present invention, it is provided that the reference parameter set and/or the image parameter set comprises
- shape parameters,
- artefacts generated by the exogenous structure and/or
- a location of the exogenous structure.
In particular, shape parameters include information about a two-dimensional or three dimensional
presentation/illustration of the exogenous structure, preferably of inner parts of the exogenous structure. For example, the shape parameter comprises dimensions of the specific exogenous structure. Further, artefacts usually generated by the specific exogenous structure and by a specific medical imaging device used for recording the medical image might support assigning the reference parameter set. Another parameter that might be helpful for assigning the reference parameter set is the location of the exogenous structure. For instance, a pacemaker is located in a thorax, and a knee prosthesis is located in a knee of the patient. It is also thinkable that several parts of the implant are located at different spots and the kind of distribution helps to identify the implant. By comparing more than one parameter, the probability for assigning the proper exogenous structure rises. Preferably, the reference parameter set and the image parameter set refer to the same kind of parameters.

In another preferred embodiment of the present invention, it is provided that for extracting an image parameter set from the medical imaging data set, a machine learning mechanism, in particular a deep-learning mechanism, or a pattern recognition mechanism, is used or performed. By using a machine learning mechanism, e.g. a deep-learning mechanism or a pattern recognition mechanism it is advantageously possible to automatically extract the image parameter set, in particular an image parameter set that can be compared and finally assigned to the reference parameter set. In particular, it is possible to extract information from the medical imaging based on information from previous recorded medical images. Preferably, the machine learning mechanism, e.g. an artificial network establishes a link between the image parameter set and the reference parameter set by the machine learning mechanism for supporting identifying the image parameter set in the medical imaging data set. Thus, it is possible to use correlations identified by the machine learning mechanism, e.g. an artificial network, in particular being trained based on previous recorded medical images, and, possibly, other data, for identifying the present recorded medical image. By using a pattern recognition mechanism, landmarks and/or classifying structures, such as a form of an object or potential artefacts, can be recognized for including into the image parameter set and comparing them to landmarks and/or classifying structures of the reference parameter set.

Preferably, it is provided that a further machine learning mechanism, in particular a further deep-learning mechanism, is used or performed for assigning the image parameter set. Thus, assigning can be supported by the further deep-learning mechanism.

In particular the further machine learning mechanism is trained for inspecting the proper orientation and/or position of the implant. Thus, the system can identify implants being implanted incorrectly in an automatic way. Preferably, the user is informed by the misalignment of the implant.

In another preferred embodiment of the present invention, it is provided that the identification is provided to an operator of the medical imaging device and/or used for configuring the medical imaging device. Thus, the operator can be advantageously informed immediately about the specific exogenous structure and/or the medical imaging device can be adapted to the new information about the exogenous parameter. It is also thinkable that recording of the medical image is automatically interrupted as soon as the exogenous structure is identified. Alternatively, the configuration of the medical imaging device is adapted during recording the medical image based on the identification. For example, configuration parameter, such as a dB/dt-value in a MRT-recording, is adapted to an admissible threshold value being assigned to the specific exogenous structure.

In particular, it is provided that the identification is displayed on a screen. The screen might be part of a workstation, a tablet or a smartphone. Preferably, the identification is visualized together with the medical imaging data set. For example, a model designation is displayed next to a location of the exogenous structure on the screen, and/or the exogenous structure is highlighted by colouring. Thus, the operator can recognize and identify exogenous structures quickly and easily. It is also thinkable that the operator is asked whether he is interested in displaying the identification as soon as the exogenous structure is identified.

According to another embodiment of the present invention, it is provided that the database is extended by incorporating a further exogenous structure and a corresponding further reference parameter set by a local medical imaging device. Thus, it is advantageously possible to add exogenous structures found in a patient to the database. As a consequence, the database can be permanently updated.

Preferably, it is provided that the reference parameter set is extended by transferring a medical image data set and/or an image parameter set to the database. Thus, the information stored in the database and forming the basis of the reference parameter sets are advantageously increased. Preferably, it is provided that the artificial network is trained by the additional medical image data sets and/or image parameter sets for extending the reference parameter set, in particular to train the artificial network by the machine learning mechanism. As a consequence of the training, a success rate for assigning the reference parameter to the image data set increases. It is also thinkable that information concerning a situation without the exogenous structure, for example after a surgery, is transferred to the database.

In another preferred embodiment of the present invention, it is provided that the database comprises an additional information set being assigned to the corresponding exogenous structure, wherein the additional information set is provided to the operator of the medical imaging device. Thus, the operator can be advantageously be supplied with additional information concerning the specific exogenous structure. The additional information preferably concerns information, in particular updated information, about malfunction caused by the exogenous structure as well as maintenance information. Thus, the operator and the patient can be updated about the current status of the exogenous structure and consequently protect the patient. It is also thinkable that the additional information comprises an alternative to the identified exogenous structure, such as a new improved model of the implant. Preferably, the additional information is stored in the database and, in particular, is updated.

In particular, it is provided that a further additional information set is provided, when a specific operator of the medical imaging device is identified. Preferably, an authentication mechanism is provided such that secret specifications or properties of exogenous structures are made available by the producer of the exogenous structure. Such a secret specification might be a special risk of an implant. Thus, the operator or the physician can use these secret specifications.
According to another embodiment of the present invention, it is provided that the database is part of a network, wherein preferably the database and the medical imaging device are in communication with each other. By using a common network, it is advantageously possible to establish a central update as well as collecting image data sets from different local medical imaging devices. Thus, the quality of assigning the proper reference parameter set to the image data set is further improved. Preferably, there is an interface for communicating between the local medical imaging device and the network. It is also thinkable that the evaluation unit is integrated into the medical imaging device, wherein the evaluation unit is configured for accessing the database, or that the evaluation unit is integrated in the database. In the case the evaluation unit is integrated in the database, the medical imaging data set is transferred to the database for extracting, and/or the image parameter set is transferred to the database for assigning the reference parameter set to the image parameter set. Furthermore, it is possible to upload further medical imaging data sets, such as a medical imaging data set after a surgery, for extending the information available in the database. In addition, it is conceivable that an incentive mechanism is incorporated to the database or network, such that uploading a further reference parameter set and a further exogenous parameter set are rewarded, for example, by using a digital currency such as bitcoins. Furthermore, the network is configured such that producers of the exogenous structures can add the further reference parameter set and the further exogenous structure via an internet platform. Thereby, it is conceivable that a license might be needed for adding further exogenous structures to the network or database.

In another embodiment of the present invention, a list of identification is provided when several reference parameter sets are assignable to the image parameter set. Preferably, a ranking is provided such that the operator gets a list of potential exogenous structures and their probability of being the recorded exogenous structure. Thus, the operator receives an overview of the potential exogenous structures and can adapt the further proceeding.

According to another aspect of the present invention, a system for identifying an exogenous structure, in particular according to one of the preceding claims, is provided, wherein the system is configured for:
- providing a database classifying exogenous structures by a reference parameter set;
- providing the medical imaging data set;
- extracting an image parameter set from the medical imaging data set; and
- assigning the reference parameter set to the image parameter set for identifying the exogenous structure.

Another aspect of the present invention is a computer program product for carrying out the steps of the method according to the present invention when the computer program product is loaded into a memory of a programmable device.

Preferably, the computer program is configured for offering the user two or more databases with models of exogenous structures or objects and/or offering the user to choose one or multiple databases for a particular case. In particular, it is provided that the computer program is configured such that a payment mechanism is included wherein the payment mechanism comprises a transfer of money from the operator to the database provider in case of a request and/or successful reference to an exogenous structures or object.

A further aspect of the present invention is a computer-readable medium on which is stored program elements that can be read and executed by a computer unit in order to perform steps of the method according to the present invention when the program elements are executed by the computer unit.

In the drawing:

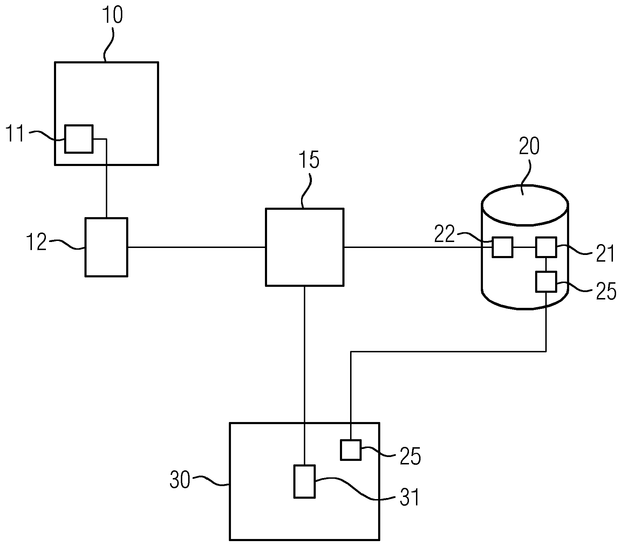

- Fig. 1: shows a flow diagram schematically illustrating a method for identifying an exogenous structure according to a preferred embodiment of the present invention.

In figure 1, a flow diagram schematically illustrating a method for identifying an exogenous structure 21 according to a preferred embodiment of the present invention is shown. In particular, the method refers to identifying exogenous structures 21, such as implants, prostheses or metal residues of a surgery, which are located inside a body, for example inside a patient. For avoiding damages, in particular at the patient, at the exogenous structure 21 and/or at the medical imaging device 10, caused by using an improper medical imaging device 10 or an improper configuration of the medical imaging device 10, it is crucial to know the specifications of the exogenous structure 21. However, many patients do not know the type and/or specification of their exogenous structure 21. By identifying the exogenous structure 21 independently, there is a chance to adapt the further treatment to the specifications of the exogenous structure 21 and to reduce the risk of damages.

Thereby, a database 20 is provided, wherein the database comprises reference parameter sets 22 being each assigned to a specific exogenous structure 21, i.e. each exogenous structure 21 is classified by a specific reference parameter set 22. This reference parameter set comprises, for example, shape parameters, such as two dimensional or three dimensional illustrations of the exogenous structure 21, in particular size data of the exogenous structure 21. Additionally or alternatively, the reference parameter set comprises information about artefacts being in general generated by the exogenous structure 21 when the exogenous structure is recorded by the respective medical imaging device 20 and/or a typical location of the exogenous structure 21, such as a chest region.

Furthermore, it is provided that a medical imaging data set 11 is provided, in particular by recording the medical imaging data set 11 using a medical imaging device 10, such as an x-ray-imaging device, a computer tomography (CT) device, a magnetic resonance tomography (MRT) device or an ultrasound scanner. For identifying the exogenous structure 21, an image parameter set 12 is extracted from the medical imaging device 11. Preferably, the image parameter set 12 is adapted to the reference parameter set 22, i.e., at least a subset of information being extracted and being compiled to the image data set 12 can be correlated to information being included in the reference parameter set 22. For example, the image data set 12 includes size data being assigned to the recorded exogenous structure 21 or information classifying an artefact. In particular, the image parameter set 12 is extracted from the medical imaging data 11 set automatically, preferably by using a machine learning mechanism, such as a deep-learning mechanism, or by using a pattern recognition mechanism.

In particular, an evaluation unit 15 is provided. Such an evaluation unit is configured for assigning the reference parameter set 22 to the image parameter set 12 for identifying the exogenous structure 21, i.e., the evaluation unit compares and/or correlates the image parameter set 12 and the reference data set 22. In particular, it is provided that the evaluation unit 15 has access to the database 20 or is part of the date base 20. Further, it is thinkable that the medical imaging data set 11 and/or the image parameter set 12 is transferred to the evaluation unit 15. Preferably, it is provided that for assigning the image parameter set 12 a further machine learning mechanism, in particular a further deep-learning mechanism, is performed. As soon as the reference data set 22 is correlated to the image data set 12 by the evaluation unit 15, the exogenous structure 21 is identified, since the exogenous structures 21 are classified in the database 20. Preferably, a corresponding identification, such as a type description of the exogenous structure 21, is provided, for example by displaying on a screen of a workstation, a tablet, a smartphone or a computer. In particular, it is provided that the identification is incorporated into the medical image data set 11 or preferably into the visualisation of medial image data set 11. For supporting an operator of the medical imaging device 10, an additional information set 25 is preferably provided, for example by visualizing the additional information on the screen together with the identification. The additional information set 25 might comprise, for example:
- thresholds which have to be considered in case of recording a medical image data set 10 of the specific exogenous structure 21 by the specific medical imaging device 10,
- information about typical malfunctions of the exogenous structure 21 or malfunctions caused by exogenous structure 21
- information about alternatives to the exogenous structure 21.

## Claims

1. Method for identifying an exogenous structure (21), in particular an implant, a prosthesis or metal residues, in a medical imaging data set (11) being recorded by a medical imaging device (10), comprising:
- providing a database (20) classifying exogenous structures (21) by a reference parameter set (22);
- providing the medical imaging data set (11);
- extracting an image parameter set (12) from the medical imaging data set (11); and
- assigning the reference parameter set (22) to the image parameter set (12) for identifying the exogenous structure (21) .

2. Method according to claim 1, wherein the reference parameter set (22) and/or the image parameter set (12) comprises
- shape parameters
- artefacts generated by the exogenous structure (21) and/or
- a location of the exogenous structure (21).

3. Method according to one of the preceding claims, wherein for extracting an image parameter set (12) from the medical imaging data set (11) a machine learning mechanism, in particular a deep-learning mechanism, or a pattern recognition mechanism is performed and/or used.

4. Method according to one of the preceding claims, wherein for assigning the image parameter set (12) a further machine learning mechanism, in particular a further deep-learning mechanism, is performed and/or used.

5. Method according to one of the preceding claims, wherein the identification (31) is provided to an operator of the medical imaging device (10) and/or used for configuring the medical imaging device (10).

6. Method according to one of the preceding claims, wherein the identification (31) is displayed on a screen (30).

7. Method according to one of the preceding claims, wherein the database (20) is extended by incorporating a further exogenous structure and a corresponding further reference parameter set by a local medical imaging device.

8. Method according to one of the preceding claims, wherein the reference parameter set (22) is extended by transferring a medical image data set (11) and/or an image parameter set (12) to the database (20).

9. Method according to one of the preceding claims, wherein the database (20) comprises an additional information set (25) being assigned to the corresponding exogenous structure (21), wherein the additional information (25) set is provided to the operator of the medical imaging device (10).

10. Method according to one of the preceding claims, wherein a further additional information set is provided, when a specific operator of the medical imaging device (10) is identified.

11. Method according to one of preceding claims, wherein the database (20) is part of a network, wherein preferably the database (20) and the medical imaging device (10) are in communication with each other.

12. Method according to one of the preceding claims, wherein a list of identification (31) is provided, when several reference parameter sets (22) are assignable to the image parameter set (12).

13. System for identifying an exogenous structure, in particular according to one of the preceding claims, wherein the system is configured for:
- providing a database (20) classifying exogenous structures (21) by a reference parameter set (22);
- providing the medical imaging data set (11);
- extracting an image parameter set (12) from the medical imaging data set (11); and
- assigning the reference parameter set (22) to the image parameter set (12) for identifying the exogenous structure (21) .

14. Computer program product for carrying out the steps of the method according to any of claims 1 to 12 when the computer program product is loaded into a memory of a programmable device.

15. A computer-readable medium on which is stored a program elements that can be read and executed by a computer unit in order to perform steps of the method according to any of the claims 1 to 12 when the program elements are executed by the computer unit.
